# EUROPEAN PATENT SPECIFICATION

(11) **EP 1 596 670 B1**
(45) Date of publication and mention of the grant of the patent: **07.05.2014**
(21) Application number: 04709679.7
(22) Date of filing: 10.02.2004
(51) Int. Cl.: A23L 1/30, A61K 31/20, A61K 31/575, A61K 31/56, A23D 9/013, A61P 9/00

(54) **OILS ENRICHED WITH DIACYLGLYCEROLS AND PHYTOSTEROL ESTERS FOR USE IN THE REDUCTION OF CHOLESTEROL AND TRIGLYCERIDES**
MIT DIACYLGLYCEROL UND PHYTOSTEROLESTERN ANGEREICHTE ÖLE UND DEREN VERWENDUNG FÜR DIE VERRINGERUNG DES CHOLESTERIN- UND TRIGLYZERIDGEHALTES IM BLUT
HUILES ENRICHIES AVEC DES DIACYLGLYCEROLES ET DES ESTERS DE PHYTOSTEROL UTILISEES DANS LA DIMINUTION DU CHOLESTEROL ET DES TRIGLYCERIDES

(30) Priority: 10.02.2003 IL 15438103; 27.03.2003 IL 15513603
(43) Date of publication of application: 23.11.2005
(73) Proprietor: Enzymotec Ltd., 36584 Kfar Baruch (IL)
(72) Inventor: PLATT, Dorit, 17906 Shimshit (IL); PELLED, Dori, 45325 Hod Hasharon (IL); SHULMAN, Avidor, 36090 Kiryat Tivon (IL)
(74) Representative: Tripoz, Inès
(86) International application number: PCT/IL2004/000131
(87) International publication number: WO 2004/069150

(56) References cited:
- WO-A-02/11550
- WO-A-03/064444
- US-A- 5 843 499
- US-A- 6 129 924
- US-A1- 2001 046 548
- US-A1- 2002 045 000
- US-B1- 6 326 050
- MUSSNER M J ET AL: "Effects of Phytosterol Ester-Enriched Margarine on Plasma Lipoproteins in Mild to Moderate Hypercholesterolemia Are Related to Basal Cholesterol and Fat Intake" METABOLISM, CLINICAL AND EXPERIMENTAL, W.B. SAUNDERS CO., PHILADELPHIA, PA, US, vol. 51, no. 2, February 2002 (2002-02), pages 189-194, XP002267083 ISSN: 0026-0495
- LING W H ET AL: "MINIREVIEW DIETARY PHYTOSTEROLS: A REVIEW OF METABOLISM, BENEFITS AND SIDE EFFECTS" LIFE SCIENCES, PERGAMON PRESS, OXFORD, GB, vol. 57, no. 3, 1995, pages 195-206, XP000995149 ISSN: 0024-3205

## Description

### Field of the Invention

The present invention relates to the use of a composition comprising a combination of diacylglycerol(s) (DAG), mainly 1,3-diacylglycerol(s), and phytosterol and/or phytostanol ester(s) (PSE) dissolved or dispersed in a fish oil, in the manufacture of nutritional supplements and orally administrable pharmaceutical preparations which are capable of reducing serum levels of both cholesterol and triglycerides, and also exhibit LDL-anti-oxidative properties, and which are suitable for the treatment and prevention of cardiovascular disease (CVD) and coronary heart disease (CHD).

### Background of the Invention

Coronary Artery Disease (like atherosclerosis) is the major cause of morbidity and mortality in the Western world and its pathogenesis involves complicated interactions between cells of the arterial wall, blood cells, and plasma lipoproteins [Ross R. (1993) Nature 362: 801-809; Glass C.K. and Witztum J.L. (2001) Cell 104:503-516]. Today, it is common knowledge that lowering cholesterol levels reduces the risk of heart attacks, strokes and other forms of atherosclerotic vascular disease. In addition, many recent studies have shown that oxidative stress is a mechanism with a central role in the pathogenesis of atherosclerosis, cancer, and other chronic diseases. In this scenario, a key role is played by macrophages in the sub-endothelial space, which are activated by oxidized low-density lipoproteins (ox-LDL). Recently, endothelial dysfunction due to oxidative stress was identified as a priming factor in the course of the development of atherosclerotic plaques.

The early atherosclerotic lesion is characterized by foam cells derived from cholesterol loaded macrophages [Gerrity R.G. (1981) Am. J. Pathol. 103:181-190; Schaffner T. et al. (1980) Am. J. Pathol. 100:57-80]. Macrophage cholesterol accumulation and foam cell formation are the hallmark of early atherogenesis and most of the cholesterol in these cells is derived from plasma low-density lipoprotein (LDL). Native LDL however, has to undergo some modifications in order to cause extensive macrophage cholesterol accumulation [Brown M.S. and Goldstein J.L. (1983) Annu. Rev. Biochem. 52:223-261; Kaplan M. and Aviram M. (1999) Clin. Chem. Lab. Med. 37:777-787; Aviram M. (1993) Atherosclerosis 98:1-9.; Steinberg D. et al. (1989) N. Engl. J. Med. 320: 915-924]. The most studied modification with a potential pathological significance is LDL oxidation [Aviram M. (1996) Europ. J. Clin. Chem. Clin. Biochem. 34:599-608; Aviram M. (1995) Isr. J. Med. Sci. 31:41-249; Chisolm G.M. and Steinberg D. (2000) Free Radic. Biol. Med. 28:1815-1826]. This modification leads to increased macrophage uptake of the modified lipoprotein, followed by cellular cholesterol accumulation that results with the formation of lipid-laden foam cells [Aviram (1996) *id ibid*.; Aviram (1995) id *ibid*.; Chisolm (2000) *id ibid*.; Aviram M. (1999) Antiox. Redox. Signal 1:585-594].

Several reports have implicated oxidative stress as the main factor triggering atherosclerosis [Heinecke, J. W. (2003) Am. J. Cardiol. 91:12A-16A; Ceconi, C. et al. (2003) Arch. Biochem. Biophys. 420:217-221; Dhalla, N. S. et al. (2000) J. Hypertens. 18:655-673]. Oxidative stress is defined as the result of an excess in free radicals (FR), which come in contact with cellular membranes and can lead to oxidative damage in biological molecules, such as lipids, carbohydrates, proteins and nucleic acids [Thomas C. E. and Aust, S. D. (1986) Ann. Emerg. Med. 15(9): 1075-83]. One of the molecules that may be attacked by FR is LDL, forming ox-LDL, whose high levels lead to atherosclerosis.

Increasing evidence in both experimental and clinical studies suggests that oxidative stress plays a major role in the pathogenesis of both types of diabetes mellitus. The possible sources for the overproduction of reactive oxygen species is widespread and include enzymatic pathways, autoxidation of glucose and the mitochondria. Abnormally high levels of these free radicals and the simultaneous decline of antioxidant defense mechanisms can lead to increased lipid peroxidation, damage of cellular organelles and enzymes and development of CVD. Thus, prevention of oxidative stress in diabetes is considered by many investigators to be a primary defense against the development of diabetic vascular disease. Moreover, some recent studies point at oxidative stress, activation of the sorbital pathway, advanced glycation endproducts (AGE), and AGE precursors, as the basic abnormalities that lead to the CVD in these patients, rather than hyperglycemia [Duckworth W.C. (2001) Curr. Atheroscler. Rep. 3:383-91; Yorek M.A. (2003) Free Radic. Res. 37:471-80; Maritim A.C. (2003) J. Biochem. Mol. Toxicol. 17:24-38.)

Paraoxonase (PON1) is a glycoprotein transported in the plasma as a component of HDL. It has been shown *in vitro* that PON1 inhibits the oxidative modification of LDL. Thus, the presence of PON1 in HDL may account for a proportion of the anti-oxidant properties of these lipoproteins [Mackness, M.I. et al. (1991) FEBS Lett. 286:152-154].

The LDL oxidation hypothesis of atherosclerosis raised an extensive investigation into the role of anti-oxidants against LDL oxidation as a possible preventive treatment of atherosclerosis. Although increased resistance of LDL to oxidation was observed after treatment with various synthetic pharmaceutical agents, an effort has been made to identify natural food products, which offer anti-oxidant defense against LDL oxidation.

Olive oil has been shown to inhibit LDL oxidation and this effect could be related to its high oleic acid content, as well as to some phenolics (hydroxytoluene, oleoropein) and phytosterols such as sitosterol [Aviram M. and Kasem E. (1993) Ann. Nutr. Metabol. 37:75-84; Visioli F. et al. (1995) Atherosclerosis 117:25-32].

In addition to LDL oxidation, a known risk factor for coronary heart disease (CHD) - the result of atherosclerosis in the coronary arteries - includes high serum LDL cholesterol concentration. There is a positive linear relationship between serum total cholesterol and LDL cholesterol concentrations, and risk of, or mortality from CHD [Jousilahtu et al. (1998) Circulation 97:1084-1094].

High concentrations of serum triacylglycerols may also contribute to CHD [Austin, M. A. (1989) Am. J. Epidemiol. 129:249-259], but the evidence is less clear. Diacylglycerols (DAG) have been shown to lower the postprandial elevation of serum triacylglycerols levels compared with triacylglycerols in healthy humans [Taguchi et al. (2000) J. Am. Coll. Nutr. 19:789-796]. Serum triglyceride (TG) concentrations after ingestion of 44 g of DAG oil were significantly low at six hours postprandially as compared to those after ingestion of 44 g of TG oil. The difference was reproducible even with low fat doses (10 and 20 g) [Robert A. Moreau et al. (2002) Progress in Lipid Research 41:457-500].

### Phytosterols and CHD

The term "phytosterols" covers plant sterols and plant stanols. Plant sterols are naturally occurring substances present in the diet as minor components of vegetable oils. Plant sterols have a role in plants similar to that of cholesterol in mammals, e.g. forming cell membrane structures. In human nutrition, both plant sterols and plant stanols are effective in lowering total plasma cholesterol levels and LDL-cholesterol.

The consumption of plant sterols and plant stanols lowers blood cholesterol levels by inhibiting the absorption of dietary and endogenously-produced cholesterol from the small intestine. The plant sterols/stanols are very poorly absorbable compounds. This inhibition is related to the similarity in physicochemical properties of plant sterols and stanols to cholesterol.

The blood cholesterol-lowering effect of plant sterols has been investigated in a large number of clinical trials involving over 2,400 subjects, using doses as high as 25 grams per day for durations as long as three years. No significant adverse effects have been observed throughout the decades of medically supervised clinical efficacy testing or the general clinical use of plant sterols. Furthermore, the drug Cytellin (primarily β-sitosterol) was prescribed for more than 20 years and had an excellent safety record.

In addition, both plant sterols and plant stanols have been subjected to rigorous toxicological evaluation. Studies on the absorption, distribution, metabolism and excretion have shown that plant sterols are poorly absorbed from the intestine (1 - 10%).

A series of human studies with vegetable oil plant sterol esters in spreads, with intakes of up to 8.6 grams of plant sterols/day for 4 weeks, has been conducted. Clinical chemistry, haematology, bacterial profiles of the gut microflora and general physical condition were evaluated. As in all other studies, no adverse effects were detected.

In the United States, a panel of independent experts has concluded that vegetable oil sterol esters, meeting appropriate food-grade specifications and produced by current good manufacturing practice (21 C.F.R. §182.1(b)), are safe for use as an ingredient in vegetable oil spreads, in amounts which do not exceed 20% of plant sterol esters. It is the Panel's opinion together with qualified experts in the field that recognized vegetable oil sterol esters as safe for use, i.e. vegetable oil sterol esters were granted the GRAS status (Generally Recognized As Safe). Based on the GRAS recognition, the US Food and Drug Administration (FDA) has cleared to use a spread containing up to 20% of plant sterol esters and another one containing plant stanol ester. Similar approvals were given in different European countries as well as in Asia and Australia.

The role of diet in the promotion or prevention of heart disease is the subject of considerable research. However, the use of naturally-occurring materials which can lower LDL-cholesterol and triglycerides levels and inhibit LDL-oxidation should be advantageous over the use of synthetic drugs.

A recent review teaches that in recent years, with the growing interest in functional foods, the use of phytosterols for reducing serum cholesterol levels has gained considerable momentum [Stark, A.H. et al. (2002) Nutrition Reviews 60(6):170-176]. This should be attributed, *inter alia,* to the esterification of phytostanol with fatty acids (stanyl esters), providing commercial scale production of phytosterol-containing foods, such as margarines. Like stanyl esters, phytosteryl esters (steryl esters) have been shown in clinical studies to consistently lower serum LDL-cholesterol (LDL-C) levels (reducing by up to about 10% or more), with no change seen in HDL-cholesterol (HDL-C) values. The review suggests that properly formulated free phytosterols and stanols may be as effective as stanyl and steryl esters in lowering LDL-C levels in humans.

WO 01/32035 teaches olive oil-based products, based on especially higher grades of olive oils (such as virgin olive oils), comprising plant stanol esters and/or plant sterol esters.

US Patent 5,843,499 discloses oil extractable from corn fiber that contains ferulate esters (phytosterol esters which are esterified to ferulic acid), in particular sitostanyl ester, which has been shown to have cholesterol-lowering activity. It is mentioned that corn fiber oil (containing about 73% fat (triacylglycerol), 8% sterol (fatty acyl) esters, 4% free sterols, 6% diacylglycerols and 6% ferulate (sterol esters)) is used as an additive to supplementary food for reducing cholesterol level.

US Patent 6,326,050 discloses a composition consisting of oil or fat, a diacylglycerol, a free phytosterol and tocopherol, dissolved or dispersed in the oil or fat. This composition plays a role in lowering blood cholesterol of hypercholesterolemic individuals. None of the above mentioned publications describes reduction of both cholesterol and triglycerides serum levels.

Olive oil, in contrast to other mentioned vegetable oils (such as corn fiber oil, table cooking oil, soybean oil, rapeseed oil, rice bran oil, and palm oil) is composed, *inter alia*, of 55 to 85% monounsaturated fatty acids (MUFA), in particular oleic acid, which contribute to the high nutritional value of this oil. There are some distinct advantages of using olive oil over other vegetable oils. Diets rich in olive oil have been shown to be more effective in lowering total cholesterol and LDL-cholesterol than conventional dietary treatments not containing high levels of MUFA [Brown M.S and Goldstein J.L. (1983) Ann. Rev. Biochem. 52:223-261].

Furthermore, olive oil is an integral ingredient of the Mediterranean diet and accumulating data suggests that it may have health benefits that include reduction of risk factors of coronary artery disease, prevention of several types of cancer, and modification of immune and inflammatory response [Brown and Goldstein (1983) *id ibid*.].

Co-owned, co-pending Israel Patent Application No. 147942 (also published as WO 03/064444) describes a composition of matter comprising diacylglycerol(s), mainly 1,3-diacylglycerols (DAG) and phytosterol and/or phytostanol ester(s) (PSE), dispersed in oil and/or fat. Mixtures of DAG and PSE are also disclosed in US 2002/ 0045000 and WO 02/11550.

In search for a combined-effect nutritional supplement and/or pharmaceutical, the inventors have now found that the said mixture of IL147942, namely a combination of diacylglycerol(s), mainly DAGs and PSEs in a fish oil, has a synergistic effect and decreases both LDL-cholesterol and triglycerides levels in the blood. This composition may further exhibit increased serum and macrophage anti-oxidative properties, and in particular LDL anti-oxidative properties, resulting in reduction of the risk for CHD and arteriovascular diseases. These novel therapeutic uses are major objects of the present invention.

It is a further object of the present invention to reduce the levels of blood cholesterol and triglycerides, and consequently the risk for CHD in mammals, for its by administration of the said composition as claimed in any one of claims 1 to 13 for its therapeutic use, which was found to decrease blood therapeutic use cholesterol levels to a greater extent compared to the combined reduction of cholesterol and triglycerides levels obtained by using each of said two ingredients (DAG and PSE) separately in oil. Such synergistic effect has not been described or demonstrated in the prior art. As mentioned, US Patent 6,326,050 refers to a combination of diacylglycerols and free phytosterols. Although it is stated in this patent that when the amount of diacylglycerols is over 80wt% a synergistic effect on lipid metabolism may be expected, no such effect is demonstrated or discussed in the patent.

It is a further object of the present invention to use said composition as a dietary/nutritional supplement (food additive) or in pharmaceutical preparation form.

These and other objects of the invention will become apparent as the description proceeds.

### Summary of the Invention

The present invention relates to a dietary nutrient (i.e. a food article) or food supplement comprising an edible composition as claimed in any one of claims 1 to 13 for its therapeutic use for reducing blood levels of both cholesterol and triglycerides and/or for lowering serum, serum LDL and macrophage oxidation levels, inhibiting the formation of foam cells and/or preventing the deleterious effects generated by lipid-induced oxidative stress, comprising a combination of DAG, mainly 1,3-DAG(s) and PSE dissolved or dispersed in a fish oil.

The fatty acid residues of the DAG in said composition may correspond to the fatty acid residues of the oil used. Exemplary residues are oleic, palmitic, palmitoleic, stearic, linoleic, linolenic, docosahexaenoic, eicosapentaenoic and eicosanoic acid residues.

The phytosterol ester(s) in said composition may be any fatty acid esters, for example but not limited to oleic and palmitic esters of stigmasterol, sitosterol, beta-sitosterol, brassicasterol, campesterol, 5-avenasterol and isomers and derivatives thereof.

In the dietary nutrient (i.e. food article) or food supplement for its therapeutic use according to the invention, said composition may further comprise conventional ingredients of nutritional compositions.

It is disclosed a composition comprised in the dietary nutrient or food supplement of the invention, the molar ratio between diacylglycerol(s) and phytosterol and/or phytostanol ester(s) is from about 1:5 to about 5: 1.

It is disclosed a dietary nutrient or food supplement, wherein the amount of diacylglycerol(s) in said composition is from 1 to 99wt%, preferably from 7 to 48wt%, and the amount of phytosterol and/or phytostanol ester(s) in said composition is from 1 to 99wt%, preferably from 5 to 50wt%.

It is disclosed a dietary nutrient or food supplement, wherein said composition consists of 15wt% DAG, mainly 1,3-diacylglycerol(s) and 25wt% total PSE dissolved or dispersed in olive oil. In an exemplary composition, the phytosterol esters consist of about 0.4wt% brassicasterol ester, 3.1wt% campesterol ester, 2.0wt% stigmasterol ester, 5.3wt% beta-sitosterol ester, 0.2wt% avenasterol ester, and the DAG contains the following fatty acid residues: oleic (65wt%), palmitic (15wt%), linoleic (10wt%) and 10wt% fatty acid residues comprising palmitoleic, stearic, linolenic and arachidic. In a particular embodiment, the said composition consists of 15wt% DAG, mainly 1,3-diacylglycerol(s) and 25wt% total phytosterol ester(s) (PSE) dissolved or dispersed in olive oil.

As referred to herein, the term dietary nutrient also relates to a food article, or a food product.

In a second aspect, the invention relates to an orally administrable pharmaceutical composition for reducing blood levels of both cholesterol and triglycerides and/or for lowering serum, serum LDL and macrophage oxidation levels, inhibiting the formation of foam cells and/or preventing the deleterious effects generated by lipid-induced oxidative stress, comprising a combination of DAG, mainly 1,3-diacylglycerol(s) and PSE dissolved or dispersed in a fish oil , and optionally further comprising pharmaceutically acceptable additives, diluents, excipients and/or carriers.

In the pharmaceutical composition of the invention, the molar ratio between diacylglycerol(s) and phytosterol and/or phytostanol ester(s) in said combination is preferably from about 1:5 to about 5: 1.

It is disclosed a pharmaceutical composition of the invention, consists of diacylglycerol(s) in an amount of from 1 to 99wt%, preferably from 7 to 48wt%, and the amount of phytosterol and/or phytostanol ester(s) in said combination is from 1 to 99wt%, preferably from 5 to 50wt%.

It is disclosed a pharmaceutical composition of the invention consists substantially of 15wt% DAG, mainly 1,3-diacylglycerol(s) and 25wt% total PSE dissolved or dispersed in olive oil. The composition exhibits an effect on the reduction of blood cholesterol and triglycerides levels. In an exemplary composition, the phytosterol esters consist of 0.4wt% brassicasterol ester, 3.1wt% campesterol ester, 2.0wt% stigmasterol ester, 5.3wt% beta-sitosterol ester and 0.2wt% avenasterol ester. The DAG(s) contain oleic acid residues (65wt%), palmitic acid residues (15wt%), linoleic acid residues (10wt%) and 10wt% other fatty acid residues, which are mainly palmitoleic, stearic, linolenic and arachidic acid residues.

The pharmaceutical composition of the invention is particularly suitable for the treatment and/or prevention of cardiovascular disorders and diseases related to the same, like coronary heart disease, atherosclerosis and cardiovascular disorders induced or manifested by other diseases such as diabetes mellitus, particularly Type II diabetes, as well as any disorder associated with lipid related oxidative stress or lipoprotein and macrophage atherogenicity.

In a further aspect, the invention relates to the therapeutic use of the said combination of DAG, mainly 1,3-diacylglycerol(s) and PSE dissolved or dispersed in a fish oil, and optionally further comprising pharmaceutically acceptable additives, diluents, excipients and/or carriers in the preparation of an orally administrable pharmaceutical composition for reducing blood levels of both cholesterol and triglycerides and/or for lowering serum, serum LDL and macrophage oxidation levels, inhibiting the formation of foam cells and/or preventing the deleterious effects generated by lipid-induced oxidative stress.

### Brief Description of the Figures

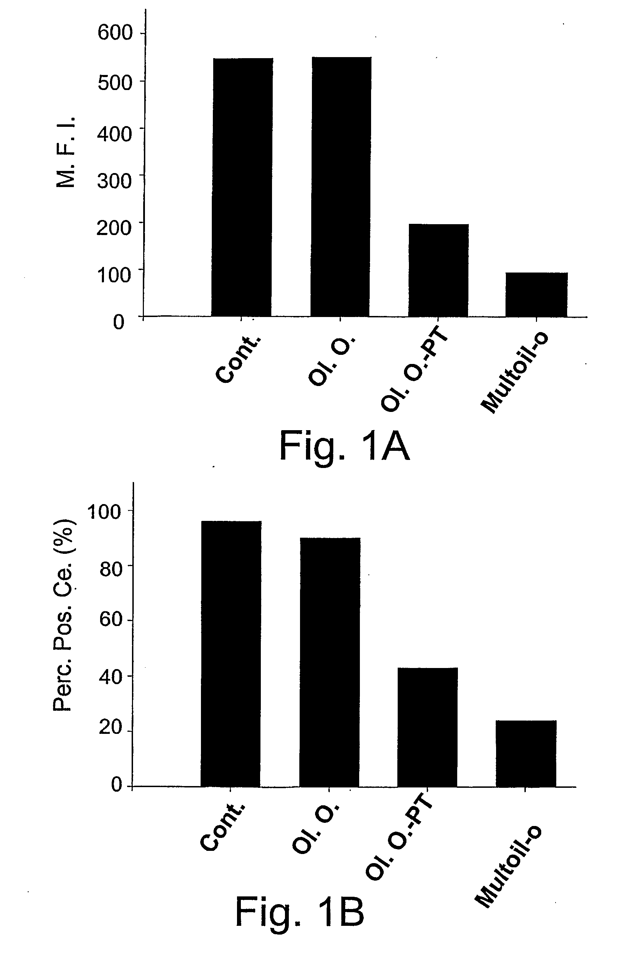

**Figure 1A****-B:** The effect of olive oil, olive oil+phytosterols, and olive MultOil on macrophage cellular peroxides content.
   Fig. 1A: Macrophage peroxide levels determined by the cells mean fluorescence (emitted by DCF) intensity.
   Fig. 1B: Macrophage peroxide levels determined by the percentage of fluorescent positive cells.
   Abbreviations: PT, Phytosterols; cont., control; Ol. O., olive oil, M.F.I, Mean Fluorescence Intensity; Perc. Pos. Ce.; Percentage of Positive Cells.
**Figure 2****:** The effect of olive oil, olive oil+phytosterols, and olive MultOil on macrophage superoxides anions release.
   Macrophage superoxide ions release was determined by the superoxide dismutase-inhibitable reduction of cytochrome C.
   Abbreviations: PT, Phytosterols; cont., control; Ol. O., olive oil; S.O. rel., superoxide release; prot., protein.
**Figure 3****:** Effect of MultOil-c and MultOil-f consumption on serum triglycerides profile.
   Abbreviations: Triglyc., Triglycerides; Plac., placebo; Can., canola.
**Figure 4****:** Effect of MultOil-c and MultOil-f consumption on serum total cholesterol profile.
   Abbreviations: Chol., Cholesterol; Plac., placebo; Can., canola.
**Figure 5****:** Effect of MultOil-c and MultOil-f consumption on serum oxidative stress.
   Abbreviations: Ser. lip. Per., serum lipid peroxides; Plac., placebo; Can., canola.
**Figure 6****:** Effect of MultOil-c and MultOil-f consumption on serum PON1 activity.
   Abbreviations: Ser. PON1 act., serum PON1 activity; Plac., placebo; Can., canola.
**Figure 7****:** Effect of MultOil-c and MultOil-f consumption on ox-LDL uptake by peritoneal macrophages.
   Abbreviations: prot., protein; Plac., placebo; Can., canola; deg., degradation; ass., association.
**Figure 8****:** Effect of MultOil-c and MultOil-f consumption on macrophage oxidative status.
   Abbreviations: Rel. Fluor. U., Relative Fluorescence unit; Plac., placebo; Can., canola.
**Figure 9****:** Effect of MultOil-c and MultOil-f consumption on PMA-induced superoxide anions release in macrophages.
   Abbreviations: S.O. rel., superoxide release; prot., protein; Plac., placebo; Can., canola.

### Detailed Description of the Invention

The following abbreviations are used along the specification:
Canola MultOil (MultOil-c): MultOil in which the base is canola oil.
CHD: Coronary heart disease
CVD: Cardiovascular disease
DAG: Diacylglycerol(s), mainly 1,3-diacylglycerols
DHA: Docosahexaenoic Acid
Fish MultOil (MultOil-f): MultOil in which the base is fish oil.
HBSS: Hanks' Balanced Salts Solution
HDL: High density lipoproteins
LDL: Low density lipoproteins
MPM: Mouse peritoneal macrophages
MultOil: A combination of diacylglycerol(s), mainly 1,3-diacylglycerol(s) and phytosterol and/or phytosterol esters in an oil and/or fat base.
Olive MultOil (MultOil-o): MultOil in which the base is olive oil.
Ox-LDL: Oxidized LDL
PBS: Phosphate Buffered Saline
PSE: Phytosterol or phytostanol ester(s).

The present inventors have used the animal model system apoE⁰ mice, in which severe hypercholesterolemia and atherosclerotic plaques are generated at an early age to evaluate the anti-atherosclerotic properties of novel edible compositions, herein referred to as MultOil-o, MultOil-c and MultOil-f, in comparison with placebo and/or with canola oil.

As mentioned above, the inventors, have found that a combination of DAGs, mainly 1,3-DAGs and PSE in a fish oil (also referred to as MultOil-f), provides an enhanced effect, by decreasing both LDL-cholesterol and triglycerides levels in the blood. This combination, and compositions comprising the same, further exhibits increased serum, serum LDL and macrophage anti-oxidative properties, as well as inhibiting the formation of foam cells and/or preventing the deleterious effects generated by lipid-induced oxidative stress, which result in reduction of the risk for CHD and arteriovascular-related diseases, like, e.g., diabetes.

Depending on the oil used as base for said combination, either if it is olive, canola or of marine origin, the combination is referred throughout the specification as MultOil-o (Olive MultOil), MultOil-c (Canola MultOil) or MultOil-f (Fish MultOil), respectively.

The invention thus mainly relates to a novel use of a combination comprising diacylglycerols and phytosterol and/or phytostanol ester(s) as an agent capable of reducing blood levels of both cholesterol and triglycerides and/or for lowering serum, serum LDL and macrophage oxidation levels, inhibiting the formation of foam cells and/or preventing the deleterious effects generated by lipid-induced oxidative stress.

It is disclosed as demonstrated in the Examples and in the Figures, the various MultOils were capable of substantially reducing the levels of blood triglycerides and cholesterol, compared to a placebo treatment, in the animal model system.

The combination used by the present invention consists essentially of phytosterol and/or phytostanol ester(s) and diacylglycerol(s), mainly 1,3-diacylglycerols, dispersed in a fish oil.

The amount of diacylglycerol(s) contained in the oil may range from about 7wt% to about 48wt% and most preferably from about 10wt% to about 22wt%.

It is disclosed that the amount of phytosterol and/or phytostanol ester(s) contained in the oil may range from 1wt% to about 99wt%, preferably from about 5wt% to about 50wt% and most preferably from about 20wt% to about 35wt%.

The diacylglycerol(s) consist substantially of 1,3-diacylglycerol(s) which mainly contain unsaturated fatty acid residues. The structure of the diacylglycerol(s) depends on the particular oil and/or fat used for dissolving or dispersing the phytosterols ester(s). It is disclosed that for example, when olive oil is used, the diacylglycerols mainly consists of 1,3-dioleyl glycerol. Generally speaking, fatty acid moieties of the DAG include oleic, palmitic, palmitoleic, stearic, linoleic, linolenic, and eicosanoic fatty acids (DAGs being 1,2-dioleyl, 1,3-dioleyl, 1,2-oleyl palmitoyl and 1,3-oleyl palmitoyl glycerols).

The phytosterol and/or phytostanol ester(s) may be any phytosterol and/or phytostanol ester. Examples of such esters are stigmasteryl oleate, stigmasteryl palmitate, sitosteryl oleate, sitosteryl palmitate, betasitosteryl oleate, and betasitosteryl palmitate.

The molar ratio of phytosterol and/or phytostanol ester(s) to diacylglycerol(s) in the oil is from about 5:1 to about 1:5 and preferably about 2:1.

The oil comprised in the composition of the invention may be any edible oil including fish oil.

The diacylglycerol(s) may be obtained by any conventional enzymatic or non-enzymatic procedure. Preferably, they are obtained by inter-esterification reaction between phytosterol(s) and triglyceride(s) present in the oil. The phytosterol and/or phytostanol ester(s) may be obtained by any conventional enzymatic or non-enzymatic procedure. Preferably, it is obtained by interesterification reaction between phytosterol(s) and/or phytostanol and triglyceride(s) present in the edible oil. A process for obtaining the combinations used by the present invention is described in detail in said IL 147942, fully incorporated herein by reference. As described in IL 147942, the composition of the present invention may be also prepared by mixing (or blending) the desired amounts of diacylglycerol(s) and phytosterol and/or phytostanol ester(s) with the oil.

As shown in the following examples, a significant effect of the tested composition in preventing and/or reducing serum ox-LDL, as well as macrophage oxidation was obtained. Thus, in addition to having an effect on reduction of blood LDL-cholesterol and triglycerides levels, the said combination, and compositions comprising the same, exhibit serum LDL and macrophage anti-oxidative properties. It is disclosed that the examples further show that olive oil, canola oil and/or fish oil MultOil preparations exhibited significant anti-oxidative properties, inhibiting the formation of foam cells, and/or preventing the deleterious effects generated by lipid-induced oxidative stress.

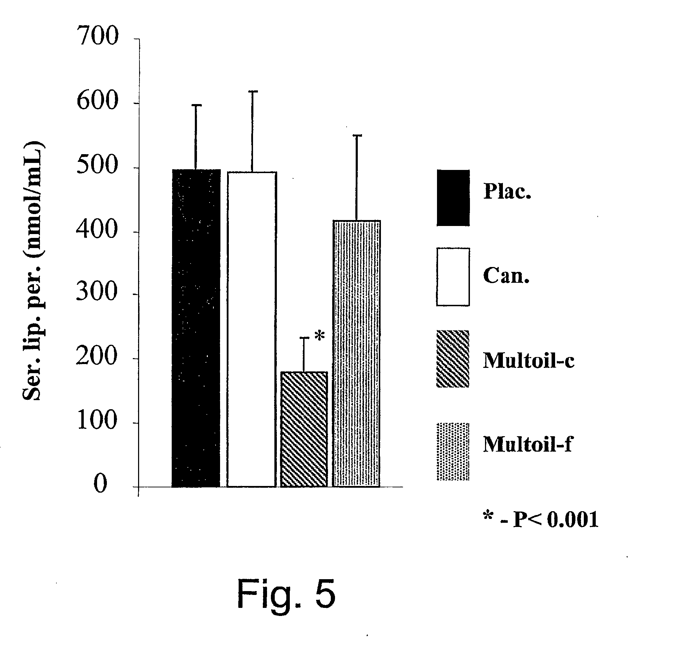

Specifically, Figures 5, 7, 8 and 9 present the results that demonstrate how the different MultOil preparations were capable of lowering the following oxidative stress parameters: serum oxidative stress, ox-LDL uptake by peritoneal macrophages, macrophage oxidative status, as well as PMA-induced superoxide anions release from macrophages.

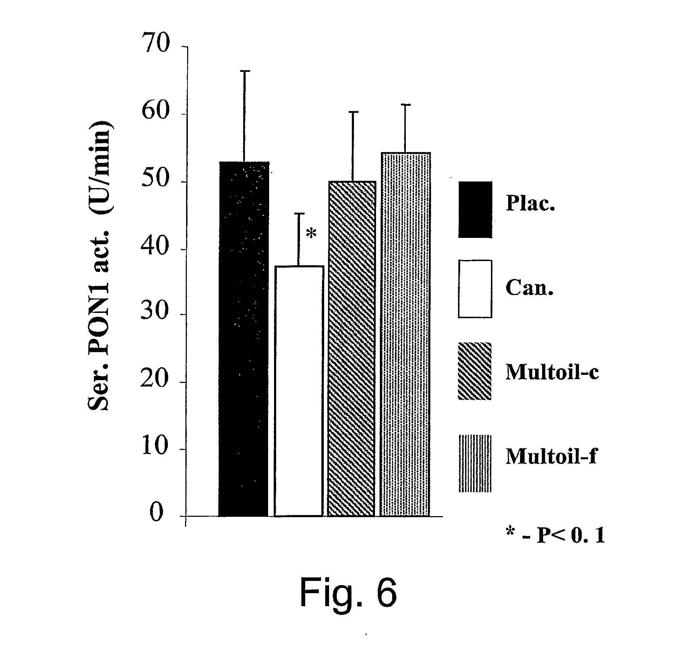

It is disclosed that the inventors have also measured PON1 activity in the serum. PON1 is an HDL-associated esterase that can eliminate ox-LDL. Interestingly, the results of Figure 6 show that while canola oil reduces PON1 activity, the MultOil combinations were able to maintain PON1 higher levels, suggesting that they may protect PON1 activity in a pro-atherosclerotic environment.

The MultOil combinations may be used *per se*, as a food article. Alternatively, it may be an ingredient of a food article or supplement, which may further optionally contain conventional additives used in the food industry, such as preserving agents, colorants, flavoring agents, fragrances, antioxidative and hardening agents, vitamins, calcium, other minerals trace elements, probiotic agents, isoflavons, caloric agents and the like.

In addition, the food supplement of the invention may be used in the manufacture of any one of functional foods, functional drinks or dietary supplements. Said food supplement may be introduced into said food, drink or dietary supplement by admixing, adding or incorporating it during manufacture thereof.

Alternatively, the combination may be comprised as the active ingredient of a pharmaceutical composition for reducing blood levels of both cholesterol and triglycerides and/or for lowering serum, serum LDL and macrophage oxidation levels, inhibiting the formation of foam cells and/or preventing the deleterious effects generated by lipid-induced oxidative stress. Pharmaceutical compositions may contain pharmaceutically acceptable additives, diluents, excipients and carriers.

The preparation of pharmaceutical compositions is well known in the art, see e.g., US Patents 5,736,519, 5,733,877, 5,554,378, 5,439,688, 5,418,219, 5,354,900, 5,298,246, 5,164,372, 4,900,549, 4,755,383, 4,639,435, 4,457,917, and 4,064,236. The combination used by the present invention may be preferably mixed with an excipient, carrier and/or diluent, and optionally, a preservative or the like pharmacologically acceptable vehicles as known in the art, see e.g., the above US patents. Examples of excipients include glucose, mannitol, inositol, sucrose, lactose, fructose, starch, corn starch, microcrystalline .cellulose, hydroxypropylcellulose, hydroxypropylmethylcellulose, polyvinylpyrrolidone and the like. Optionally, a thickener may be added, such as a natural gum, a cellulose derivative, an acrylic or vinyl polymer, or the like.

The pharmaceutical composition is preferably provided in liquid, solid or semi-solid form. The liquid preparation is provided preferably as aqueous suspension, oil suspension or microcapsule composition. A semi-solid composition is provided preferably as hydrous or oily gel or cream.

Tablets, hard tablets, capsules, and particularly, soft gelatin capsules containing the combination of the invention may be preferred, either as dietary supplements or as pharmaceutical dosage forms. In essence, any pharmaceutical dosage form suitable for oral administration may be used for delivering the combination of the invention.

It is disclosed that the dosage of the MultOil combination may depend upon the condition to be treated, the patient's age, sex and bodyweight, and will be determined by the attending physician or dietician. A preferred dosage for an adult may be from about 4 to about 6g of MultOil per day, preferably 5g, which shall comprise approximately 1300 mg of PSEs and 800mg of DAGs.

It is disclosed a method of treating and/or preventing conditions related to any one of high cholesterol and triglycerides blood levels, serum oxidative stress, ox-LDL uptake by macrophages, macrophage oxidative status, foam cells formation and lipid-induced oxidative stress, said method consisting of orally administering a therapeutically effective dosage of the food supplement or compositions thereof to a subject in need. Consequently, the method is also effective for the treatment of cardiovascular disorders, coronary heart disease, atherosclerosis, as well as cardiovascular disorders induced or manifested by other diseases such as diabetes mellitus, particularly Type II diabetes.

Alternatively, such conditions are to be treated by consumption of the dietary products in accordance with the invention.

It is disclosed a method for improving health, consisting of administering a therapeutically effective dosage of the dietary nutrient of the invention, or the pharmaceutical composition thereof to a subject in need:

The present invention is defined by the claims, the contents of which are to be read as included within the disclosure of the specification.

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following Examples are representative of techniques employed by the inventors in carrying out aspects of the present invention.

### Examples

### Materials:

- Olive oil: commercial extra virgin olive oil, manufactured by Meshek Eger (Yokeneam HaMoshava, Israel).
- Canola oil: commercial canola oil, manufactured by Shemen Taasiot (Haifa, Israel).
- Fish oil: commercial fish oil, manufactured by Pronova (Lysaker, Norway).

**Table 1: Composition of the oils used in the examples**

| **Oil components% (w/w)** | **Canola MultOil** | **Olive MultOil (Enzymotec FG S7/1.75)** | **Fish MultOil** | **Olive oil enriched with phytosterol esters** |
|---|---|---|---|---|
| | | | | |
| Phytosterol esters | 26 | 28.5 | 22.12 | 18 |
| Monoglycerides | 2.1 | 1.48 | 4.72 | 0.31 |
| Diglycerides | 14.9 | 14.62 | 20.02 | -0.81 |
| Triglycerides | 46.9 | 48.9 | 40.3 | |
| Free sterols | 3.1 | 1.5 | 5.2 | 0.2 |
| FFA | 7 | 5 | 5 | 3 |
| Glycerol | N.D. | N.D. | 2.6 | N.D. |
| Brassicasterol | 0.54 | 0.46 | 0.82 | N.D. |
| Campasterol | 5.43 | 4.58 | 4.93 | 0.009 |
| Stigmasterol | 2.84 | 3.86 | 3.25 | 0.00142 |
| Betasitosterol | 8.9 | 8.41 | 8.1 | 0.166 |

All materials were protected from light and odorous products at a temperature not exceeding 25°C.

### Methods

### Free radical scavenging capacity

The free radical-scavenging capacity of olive oil, olive oil+phytosterols, and Olive Multoil was analyzed by the DPPH assay. DPPH (1,1-diphenyl-2-picryl-hydrazyl) is a radical-generating substance that is widely used to monitor the free radical scavenging abilities (the ability of a compound to donate an electron) of various anti-oxidants [Belinky, P. A. et al. (1998) Free Radic. Biol. Med. 24: 1419-29]. The DPPH radical has a deep violet color due to its impaired electron, and radical scavenging can be followed spectrophotometrically by the loss of absorbance at 517 nm, as the pale yellow non-radical form is produced. 15 µl from stock solution of each sample were mixed with 1mL of 0.1 mmol DPPH/L in ethanol and the change in optical density at 517nm was continuously monitored.

### Isolation of mouse peritoneal macrophages

Mouse peritoneal macrophages (MPM) were harvested from the peritoneal fluid of the E° mice (15-25g) 4 days after intraperitoneal injection of thioglycolate (24 g/L) in saline (3 mL). Cells (10-20x10⁶/mouse) were washed 3 times with PBS and re-suspended to 10⁶/mL in DMEM containing 5% fetal calf serum (heat-inactivated at 56°C for 30 min), 100 U penicillin/mL, 100 µg streptomycin/mL, and 2 mM glutamine. The cell suspension was plated into culture dishes and incubated in a humidified incubator (5% CO₂, 95% air) for 2 hours. The dishes were washed once with DMEM to remove non-adherent cells, and monolayers were incubated under similar conditions for 18 hours. Mouse peritoneal macrophages were isolated from 6 mice from each group, pooled and analyzed in duplicate or triplicate for each assay.

### Macrophage superoxide release

The production of superoxide anion (O2-) by mouse peritoneal macrophages was measured as the superoxide dismutase-inhibitable reduction of cytochrome C [Yanagitani Y. et al. (1999) Hypertension 33:335-9]. Cells (1x10⁶/well) were incubated in 1 mL of HBSS containing acetyl cytochrome C (80 µmol/L). Superoxide production by the cells was stimulated by the addition of phorbol myristate acetate (0.5µg/mL) for 1 hour. To some control samples, superoxide dismutase (SOD, 30 mg/L) was added. The amount of superoxide release was determined in the medium and was expressed as nmoles of superoxides/mg cell protein, using an extinction coefficient of E₅₅₀=21 mmol/L⁻¹cm⁻¹.

### Macrophage peroxide content

Cellular peroxide levels were determined by flow cytometry using Dichlorofluorescin-diacetate (DCFH-DA) [Goupy, P. et al. (2003) Fr. Journal of Agricultural and Food Chemistry 51(3):615-622]. DCFH-DA is a non-polar dye that diffuses into the cells. In the cells it is hydrolyzed into the nonfluorescent derivate 2',7'-DCFH, which is polar and trapped within the cells. Under oxidative stress, DCFH is oxidized to DCF (2',7'-dichlorofluorescein) , which is a fluorescent compound. Peritoneal macrophages (2x10⁶) were incubated with 2.5x10⁻⁵ mol/L DCFH-DA for 30 minutes at 37°C. Reaction was stopped by washes with PBS at 4°C. Cellular fluorescence was determined with a flow cytometry apparatus (FACS-SCAN, Becton Dickinson, San Jose, CA, USA). Measurements were done at 510 to 540 nm after excitation of cells at 488 nm with an argon ion laser.

### Serum lipids profile

Serum samples were analyzed for their lipid profile including total cholesterol and triglycerides, by using commercially available kits (Roche Diagnostics, Penzberg, Germany), [Rosenblat, M. et al. (2002) Clin. Chem. Lab. Med. 40:9-14].

### Serum lipids peroxidation

Serum was diluted 1:4 in PBS. Serum susceptibility to oxidation was determined by incubating serum sample with 100mM of the free radical generating compound, 2'-2'-azobis 2'-amidinopropane hydrochloride (AAPH), which is an aqueous soluble azo compound that thermally decomposes to produce peroxyl radicals at a constant rate. The formation of thiobarbituric reactive substances (TBARS) and of lipid peroxides was measured and compared to serum that was incubated under similar conditions, but without AAPH.

### PON1 activity measurements

PON 1 activity in serum was determined by measuring arylesterase activity, using phenylacetate as the substrate. Initial rates of hydrolysis were determined spectrophotometrically at 270nm. The assay mixture included 1.0mM phenylacetate and 0.9mM CaCl₂ in 20 mM Tris HCl, pH 8.0. Non-enzymatic hydrolysis of phenylacetate was subtracted from the total rate of hydrolysis. The E₂₇₀ for the reaction was 1,310 M⁻¹ cm⁻¹. One unit of arylesterase activity is equal to 1µmol of phenylacetate hydrolyzed/min/ml. Purified enzyme has nearly 2000 units of arylesterase activity per mg protein.

### Macrophage oxidative status

Cellular oxidative stress was examined in DCF-loaded macrophages by flow-cytometry using the conversion of non-fluorescent DCFH-DA to its fluorescent counterpart DCF as an index.

### Macrophase-mediated oxidation of LDL

MPM were incubated with LDL (100µg of protein/mL) for 18 hours, under oxidative stress (in the presence of 2 µmol/L of CuSO₄), after which the extent of LDL oxidation was determined by the TBARS assay.

### Macrophage uptake of oxidized LDL

MPM were incubated with ¹²⁵I-labeled oxidized LDL (10 µg of protein/ml), and lipoprotein cell-association and degradation by these cells was determined. Lipoprotein cellular degradation was measured in the collected medium as the trichloroacetic acid (TCA)-soluble, non-lipid radioactivity, which was not due to free iodide. Lipoprotein degradation in a cell-free system was measured under identical conditions, and was subtracted from the total degradation. The remaining cells were washed three times with cold PBS and dissolved in 0.1 N

NaOH for protein and cell-associated lipoproteins Determination.

### Statistical analyses

Student t-test was used for statistical analysis of the results.

### Comparative Example 1

### Antioxidative effect of olive oil, olive oil+Phytosterols and olive Multoil against macrophage lipid peroxidation in E^{o} mice

As mentioned above, oxidative stress is involved in the pathogenesis of atherosclerosis. Atherosclerosis is associated with lipids peroxidation of plasma LDL and in arterial cells, including macrophages [Aviram M. (2000) Free. Radic. Res. 33:S85-97; Aviram M. and Fuhrman B. (1998) Mol. Cell. Biochem. 188:149-159]. Under oxidative stress, macrophage peroxide levels become increased, and macrophages generate reactive oxygen species, leading to their increased atherogenicity [Plump A.S. et al. (1992) Cell 71:343-353].

The apolipoprotein E deficient (E⁰) mice are widely used as an animal model for atherosclerosis as they develop severe hypercholesterolemia and atherosclerotic lesions on a chow diet. Moreover, in E⁰ mice, accelerated atherosclerosis is associated with increased lipid peroxidation of plasma lipoproteins and arterial cells [Hayek T. et al. (1994) Biochem. Biophys. Res. Commun. 201:1567-1574; Keidar S. (1998) Life Sci. 63:1-11].

Angiotensin II (Ang-II), a vasoconstrictor produced by the renin-angiotensin system, has been implicated in atherosclerosis. Ang-II activates macrophage NAD(P)H-oxidases, leading to increased macrophage lipid peroxidation [R,ajagopalan S. et al. (1996) J. Clin. Invest. 97:1916-1923; Johnston R.B. Jr. (1984) Methods Enzymol. 105:365-9].

In the present comparative Example, the anti-oxidative effect of three preparations of olive oil, designated olive oil+Phytosterols, olive MultOil and Olive oil, against macrophage oxidative stress was analyzed.

The following oil samples were checked (all diluted in water 1/2 vol./vol., stock solution):
1. Olive oil+Phytosterols
2. Olive MultOil
3. Olive oil

The antioxidative effect against macrophage oxidative stress of Olive MultOil in comparison to olive oil+Phytosterols and Olive oil, was analyzed by two parameters: (i) the ability to decrease macrophage peroxide content; and (ii) macrophage ability to release superoxide ions.

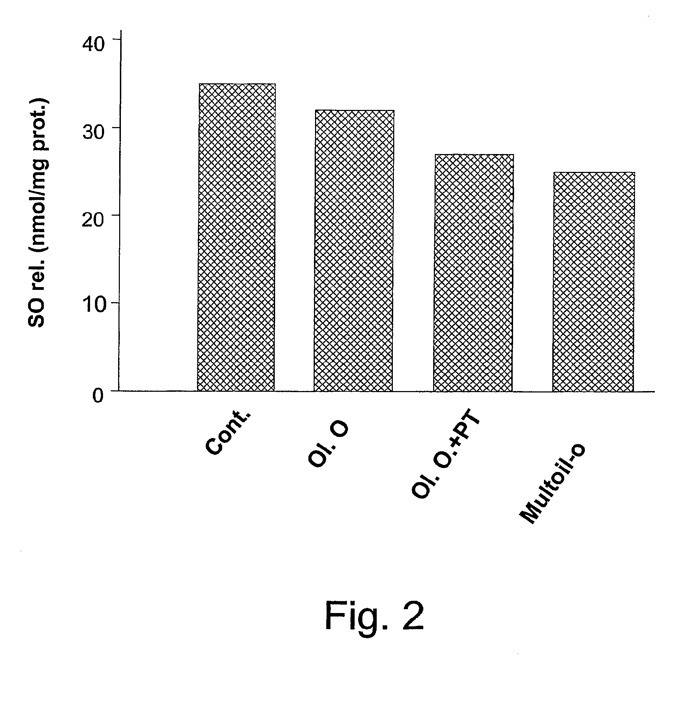

Mouse peritoneal macrophages were incubated with 50 µl of stock solution/ml of either olive oil+Phytosterols, Olive MultOil and Olive oil for 15 min, followed by a further incubation for 1 hour with Angiotensin II (10⁻⁷ M) to induce oxidative stress. Control cells were incubated with Angiotensin II alone. Macrophages were then analyzed for their peroxides content using the DCFH assay and for their ability to release superoxide ions (Fig. 2A, B).

### 1) Effect of olive oil+Phytosterols, Olive Multoil and Olive oil on macrophage peroxide content

Pre-incubation of the macrophages with both olive oil+phytosterols and Olive MultOil but not with olive oil alone reduced the macrophage peroxide content compared to control macrophages incubated with Angiotensin II alone. The macrophage lipid peroxides content, using the DCFH assay, is assessed by two parameters; first, the mean fluorescence intensity emitted by DCF and second, the percentage of cells that are positive for fluorescence emission. Preincubation of macrophages with 50 µl/ml of olive oil+phytosterols or Olive MultOil led to a reduction of 83% and 64% in macrophage mean fluorescence intensity compared to control cells, whereas olive oil at the same concentration had no effect on the macrophage mean fluorescence intensity compared to control cells (Fig. 2A). Similarly, preincubation of macrophages with 50 µl/ml of olive oil+Phytosterols or Olive MultOil led to a reduction of 74% and 55% in percentage of positive cells for fluorescence compared to control cells, whereas *olive oil* at the same concentration had no effect on the percentage of positive cells for fluorescence compared to control cells (Fig. 2B).

### 2) Effect of olive oil+Phytosterols, Olive MultOil and Olive oil on macrophages superoxides ions release

Mouse peritoneal macrophages isolated from E⁰ mice were pre-incubated with 50 µl/ml of either olive MultOil, olive oil+Phytosterols or olive oil alone for 15 minutes followed by a further incubation for 1 hour with Angiotensin II (10⁻⁷ M) to induce oxidative stress. Control cells were incubated with Angiotensin II alone.

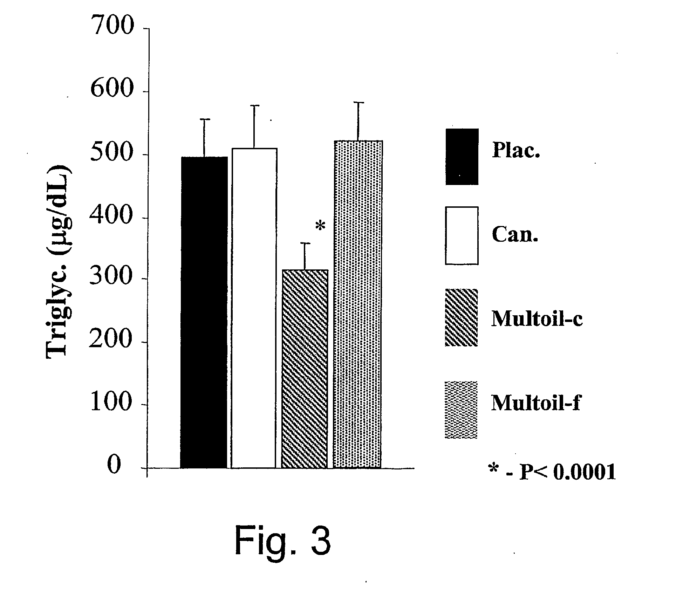

All three olive oils preparations analyzed in the present study inhibited to some extent, macrophage superoxide release induced by Angiotensin II. However, Olive MultOil and olive oil+Phytosterols were significantly more potent than olive oil alone. Pre-incubation of macrophages with 50µl/ml of Olive MultOil, olive oil+ Phytosterols or olive oil alone led to a reduction of 29%, 23% and only 9% respectively in macrophage superoxides anions release, compared to control cells incubated with Angiotensin II alone (Fig. 3).

It is disclosed that olive oil enriched with phytosterols, in particular Olive MultOil, exhibited significant anti-oxidative properties against macrophage lipid peroxidation. In contrast, whereas olive oil alone did not exhibit any effect. Most importantly, the Olive MultOil was more potent than the olive oil+phytosterols preparation in its ability to reduce macrophage peroxide content and macrophage superoxide release.

It is disclosed that these results suggest that olive oil and the additional components (phytosterols and diglycerides) can bind and internalize into the macrophages. In addition, olive oil enrichment with phytosterols enables the preparation of the invention to inhibit cellular oxidative systems (such as the NADPH oxidase and/or lypoxygenases) or to activate cellular anti-oxidant systems (such as the glutathione or superoxide dismutase systems). Furthermore, the addition of DAG to the olive oil+phytosterol preparation (resulting in Olive MultOil) led to an additional antioxidative effect towards macrophage lipid peroxidation. The inventors thus speculate that DAG, which participates in numerous intracellular signal transduction pathways, could further affect the above cellular oxidative/antioxidative systems which are involved in Angiotensin II-mediated cellular oxidative stress, expressed as macrophage lipid peroxidation and superoxide release.

### Example 2

The effect of MultOil-c and MultOil-f on the atherogenicity of lipoproteins and macrophages, and on atherosclerosis development in the atherosclerotic apolipoprotein E deficient (E°) mice model was investigated. Apolipoprotein E deficient (apoE° ) mice at 8 weeks of age, were assigned randomly to the following groups (5 mice each) as described below. The mice received regular chow diet, and in addition, they were fed (via gavage) the following, once every three days:

### Group I:

1. Placebo group: did not receive any addition of oil.
2. Canola oil group (control): were fed with 60µl of canola oil.
3. MultOil-c group: were fed with 60 µl of MultOil-c.

### Group II:

1. Placebo group: did not receive any addition of oil.
2. MultOil-f were fed with 60 µl of MultOil-f.

Each mouse consumed approximately 5 mL of water/day, and 5 g of chow/day.

### Oil Preparation for feeding

The amount of MultOil-c and MultOil-f fed to the mice were based on the following:

The recommended phytosterols dosage for humans is 1.5 gr of phytosterols/day. Based on 18.1% phytosterols in each sample, the dosage for MultOil-c and MultOil-f for humans is therefore 1.5/0.18=8.33 gr/day/person. For mice, the body weight should be taken into consideration (60,000gr human body weight/20gr mouse body weight=3000), thus the daily dosage for mouse is 8.33gr/3000=2.78mg/day/mouse, which is equal to 2.78/0.93=2.99 mL/day/mouse. Since the experiment is done for a limited period, the dosage used was 5-fold higher. Thus, each mouse was administered 15mL of oil/day (60mL/4 days/mouse).

At the end of the experimental period, blood samples were collected from all mice for serum separation and analyses. Within each experimental group, the blood sample of each mouse was analyzed individually. The following parameters were analyzed in the serum:
1. Determination of lipids, including total cholesterol and triglycerides levels.
2. Determination of serum oxidative status.
3. Determination of paraoxonase, measured as arylesterase activity.

MPM were harvested prior to removal of the heart and aorta. The mice were anesthetized with ethyl ether in a local nasal container. The heart and entire aorta were rapidly removed from all mice for histopathological analyses of aortic atherosclerotic lesions.

The experimental protocol (No. IL-066-10-2001) was approved by the Animal Care and Use Committee of the Technion Israel Institute of Technology (Haifa, Israel).

Figure 3 shows that the consumption of MultOil-c demonstrated a remarkable and significant reduction in the levels of triglycerides in the serum (36%), in comparison with placebo (p<0.001).

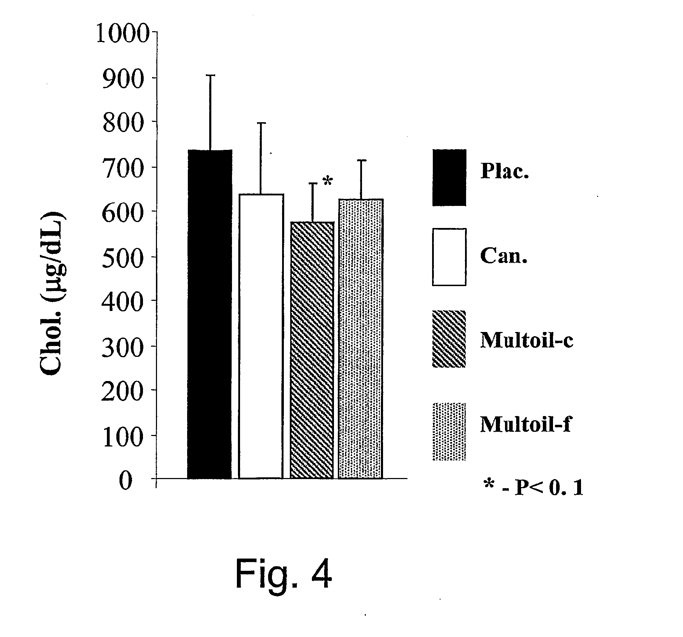

Similarly, Figure 4 shows that particularly MultOil-c, but also MultOil-f demonstrated a tendency to reduce total cholesterol levels in the serum (p<0.1).

Figure 5 shows that MultOil-c treatment resulted in a drastic and highly significant (p<0.001) reduction of the serumsusceptibility to AAPH-induced oxidation by 63% (in comparison to placebo). MultOil-f demonstrated a similar tendency, reducing lipid peroxides by 16% in comparison with placebo.

Figure 6 shows an interesting result. Whereas canola oil consumption induced a significant reduction in the levels of serum PON1 activity (p<0.1), which may be detrimental for atherosclerosis [Mackness, B. *et al.* [2003] *Circulation* **107**:2775-9], consumption of MultOil-c or -f restored PON1 activity, to levels comparable of that of untreated (Placebo group) mice. Thus, consumption of MultOil-c and MultOil-f is beneficial for maintaining effective levels of PON1 activity.

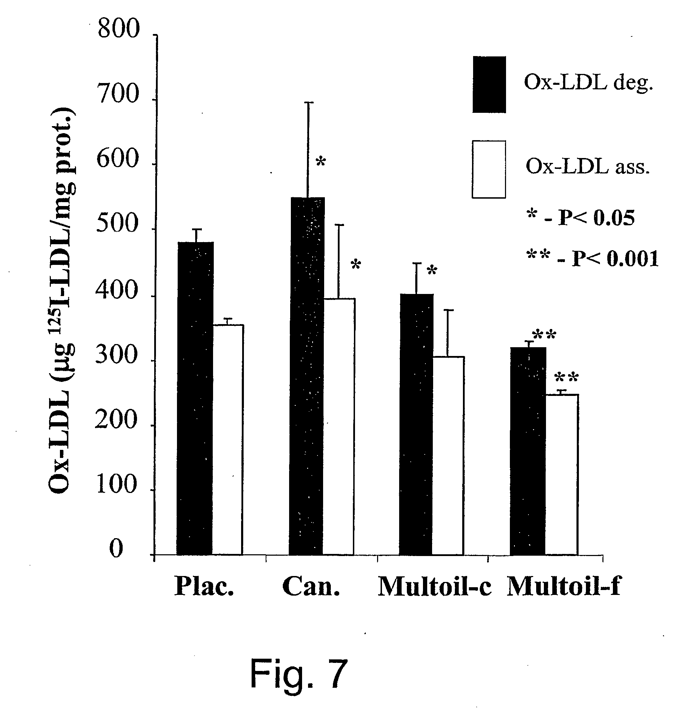

Figure 7 demonstrates that consumption of MultOil-c caused reduction of ox-LDL association (16%) and degradation (14%) (p<0.05), resulting in increased MPM abilities to sustain ox-LDL, which can be correlated to a decreased oxidation status, and to a larger extent, MultOil-f displayed a similar effect, also causing reduction of ox-LDL association (34%) and degradation (30%) (p<0.001). In contrast, canola oil consumption resulted in a slight increased (p value<0.05) of both ox-LDL association and degradation (4% and 11%, respectively, in comparison to placebo).

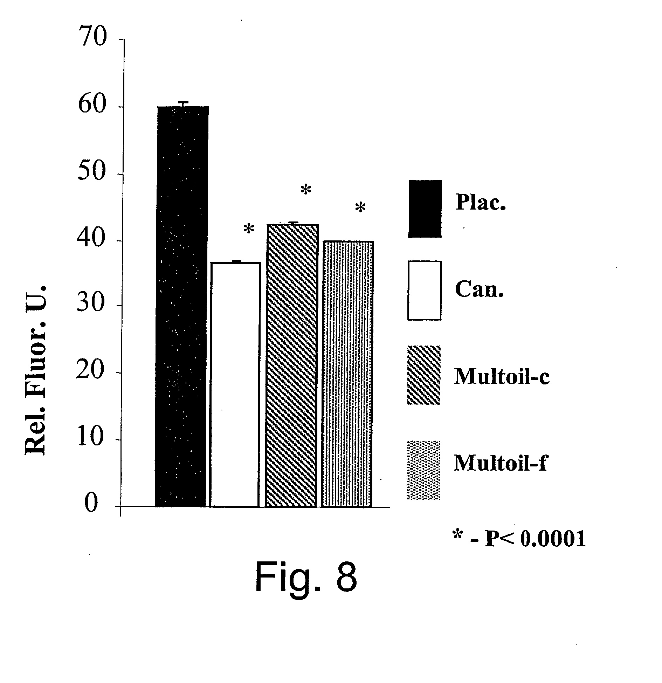

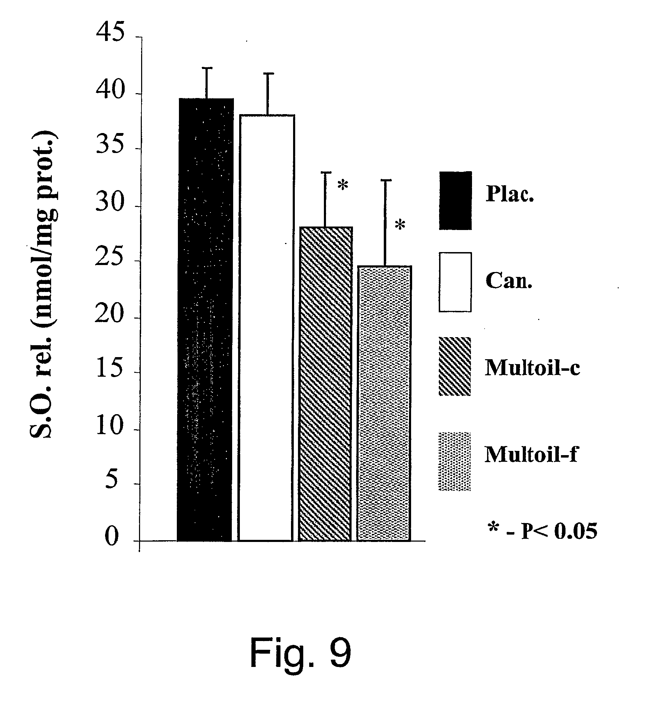

Figure 8 shows that consumption of either MultOil-c or MultOil-f significantly reduced the oxidative status of E⁰ mice macrophages (p<0.0001). MultOil-f reduced macrophages oxidative status by 34% in comparison to placebo, while MultOil-c reduced it by 29% in comparison to placebo. Thus, both MultOil-f and MultOil-c are effective in reducing the oxidative status of macrophages. Consistent with these results, in Figure 9, the inventors show that, similarly, consumption of either MultOil-f (according to the present invention) or MultOil-c (which is not comprised within the present invention) also significantly reduced the PMA-induced release of superoxide anions in macrophages (p<0.05).

## Claims

1. An edible composition for use in the treatment and prevention of cardiovascular disease and coronary heart disease,
said edible composition comprising a combination of diacylglycerol(s) (DAG), mainly 1,3-diacylglycerol(s), and phytosterol and/or phytostanol ester(s) (PSE) dissolved or dispersed in a fish oil,
wherein the amount of PSE in the oil is from 20wt% to 99wt%.

2. The edible composition according to claim 1, wherein the amount of PSE in the oil is from 20wt% to 50wt%.

3. The edible composition according to claim 2, wherein the amount of PSE in the oil is from 20wt% to 35wt%.

4. The edible composition according to any one of the preceding claims, wherein the amount of DAG in the oil is from 7wt% to 48wt%.

5. The edible composition according to the claim 4, wherein the amount of DAG in the oil is from 10wt% to 22wt%.

6. The edible composition according to any one of the preceding claims, wherein the diacylglycerol(s) and the phytosterol and/or phytostanol ester(s) are obtained by interesterification reaction between phytosterol(s) and/or phytostanol and triglyceride(s) present in the oil.

7. The edible composition according to any one of the preceding claims, wherein the fatty acid residues of the DAG correspond to the fatty acid residues of the oil used, such as oleic, palmitic, palmitoleic, stearic, linoleic, linolenic, eicosanoic, docosahexaenoic (DHA) and eicosapentaenoic (EPA) acid residues.

8. The edible composition according to any one of the preceding claims, wherein the phytosterol ester(s) is/are fatty acid esters of stigmasterol, sitosterol, beta-sitosterol, brassicasterol, campesterol, 5-avenasterol and isomers and derivatives thereof.

9. The edible composition according to any one of the preceding claims, wherein the molar ratio between diacylglycerol(s) and phytosterol and/or phytostanol ester(s) in said composition is from 1:5 to 5:1.

10. The edible composition according to claim 9, wherein the molar ratio between phytosterol and/or phytostanol ester(s) to diacylglycerol(s) in said composition is 2:1.

11. The edible composition according to any one of the preceding claims, wherein said composition consists of 15wt% DAG, mainly 1,3-DAG(s) and 25wt% total PSE(s) dissolved or dispersed in a fish oil.

12. The edible composition according to any one of the preceding claims, wherein the edible composition is an orally administrable pharmaceutical composition.

13. The edible composition according to claim 12, wherein the orally administrable pharmaceutical composition further comprises pharmaceutically acceptable additives, diluents, excipients and/or carriers.

14. A food article or food supplement which comprises an edible composition according to any one of the claims 1-13 for use in the treatment and prevention of cardiovascular disease and coronary heart disease.

15. A food article or food supplement according to claim 14, wherein the edible composition further comprises conventional ingredients of nutritional compositions.

## Patentansprüche

1. Essbare Zusammensetzung zur Verwendung bei der Behandlung und der Vorbeugung von Herz-Kreislauf-Erkrankungen und koronaren Herzerkrankungen,
wobei die essbare Zusammensetzung eine Kombination aus Diacylglycerol(en) (DAG), hauptsächlich 1,3-Diacylglycerol(en), und Phytosterol- und/oder Phytostanolester(n) (PSE), gelöst oder dispergiert in einem Fischöl, umfasst,
wobei die Menge von PSE im Öl von 20 Gew% bis 99 Gew% beträgt.

2. Essbare Zusammensetzung nach Anspruch 1, wobei die Menge von PSE im Öl von 20 Gew% bis 50 Gew% beträgt.

3. Essbare Zusammensetzung nach Anspruch 2, wobei die Menge von PSE im Öl von 20 Gew% bis 35 Gew% beträgt.

4. Essbare Zusammensetzung nach einem der vorhergehenden Ansprüche, wobei die Menge von DAG im Öl von 7 Gew% bis 48 Gew% beträgt.

5. Essbare Zusammensetzung nach Anspruch 4, wobei die Menge von DAG im Öl von 10 Gew% bis 22 Gew% beträgt.

6. Essbare Zusammensetzung nach einem der vorhergehenden Ansprüche, wobei der/die Diacylglycerol(e) und der/die Phytosterol- und/oder Phytostanolester durch eine Umesterungsreaktion zwischen dem/den Phytosterol(en) und/oder Phytostanol und Triglycerid(en), die im Öl vorhanden sind, erhalten werden.

7. Essbare Zusammensetzung nach einem der vorhergehenden Ansprüche, wobei der Fettsäurereste von DAG den Fettsäureresten der verwendeten Öle wie z.B. Olein-, Palmitin-, Palmitolein-, Stearin-, Linol-, Linolen-, Arachin-, Docosahexan- (DHA)und Eicosapentaen (EPA)-Säureresten entsprechen.

8. Essbare Zusammensetzung nach einem der vorhergehenden Ansprüche, wobei der/die Phytosterolester Fettsäureester von Stigmasterol, Sitosterol, Beta-Sitosterol, Brassicasterol, Campesterol, 5-Avenasterol und Isomeren und Derivaten davon ist/sind.

9. Essbare Zusammensetzung nach einem der vorhergehenden Ansprüche, wobei das Molverhältnis zwischen Diacylglycerol(en) und Phytosterol- und/oder Phytostanolester(n) in der Zusammensetzung zwischen 1:5 und 5:1 liegt.

10. Essbare Zusammensetzung nach Anspruch 9, wobei das Molverhältnis zwischen Phytosterol- und/oder Phytostanolester(n) zu Diacylglycerol(en) in der Zusammensetzung 2:1 ist.

11. Essbare Zusammensetzung nach einem der vorhergehenden Ansprüche, wobei die Zusammensetzung 15 Gew% DAG, hauptsächlich 1,3-DAG(s) und 25 Gew% gesamte PSE(s) umfasst, gelöst oder dispergiert in einem Fischöl.

12. Essbare Zusammensetzung nach einem der vorhergehenden Ansprüche, wobei die essbare Zusammensetzung eine oral verabreichbare pharmazeutische Zusammensetzung ist.

13. Essbare Zusammensetzung nach Anspruch 12, wobei die oral verabreichbare pharmazeutische Zusammensetzung weiter pharmazeutisch akzeptable Zusatzstoffe, Verdünnungsmittel, Trägerstoffe und/oder Trägersubstanzen umfasst.

14. Nahrungsmittelartikel oder Nahrungsmittelzusatz, der eine essbare Zusammensetzung nach einem der Ansprüche 1 - 13 umfasst, zur Verwendung bei der Behandlung und der Vorbeugung von Herz-Kreislauf-Erkrankungen und koronaren Herzerkrankungen.

15. Nahrungsmittelartikel oder Nahrungsmittelzusatz nach Anspruch 14, wobei die essbare Zusammensetzung weiter herkömmliche Bestandteile von Nahrungszusammensetzungen umfasst.

## Revendications

1. Composition comestible pour une utilisation dans le traitement et la prévention de maladie cardio-vasculaire et de maladie coronarienne,
ladite composition comestible comprenant une combinaison de diacylglycérol(s) (DAG), principalement un/des 1,3-diacylglycérol(s), et d'ester(s) de phytostérol(s) et/ou de phytostanol (PSE) dissous ou dispersés dans une huile de poisson,
dans laquelle la quantité de PSE dans l'huile se trouve dans la plage allant de 20% en poids à 99% en poids.

2. Composition comestible selon la revendication 1, dans laquelle la quantité de PSE dans l'huile se trouve dans la plage allant de 20% en poids à 50% en poids.

3. Composition comestible selon la revendication 2, dans laquelle la quantité de PSE dans l'huile se trouve dans la plage allant de 20% en poids à 35% en poids.

4. Composition comestible selon l'une quelconque des revendications précédentes, dans laquelle la quantité de DAG dans l'huile se trouve dans la plage allant de 7% en poids à 48% en poids.

5. Composition comestible selon la revendication 4, dans laquelle la quantité de DAG dans l'huile se trouve dans la plage allant de 10% en poids à 22% en poids.

6. Composition comestible selon l'une quelconque des revendications précédentes, dans laquelle le/les diacylglycérol(s) et l'ester/les esters de phytostérol(s) et/ou de phytostanol sont obtenus par une réaction d'interestérification entre le/les phytostérol(s) et/ou le phytostanol et le/les triglycéride(s) présents dans l'huile.

7. Composition comestible selon l'une quelconque des revendications précédentes, dans laquelle les résidus d'acide gras du/des DAG correspondent aux résidus d'acide gras de l'huile utilisée, tels que les résidus d'acides oléique, palmitique, palmitoléique, stéarique, linoléique, linolénique, eicosanoïque, docosahexaénoïque (DHA) et eicosapentaénoïque (EPA).

8. Composition comestible selon l'une quelconque des revendications précédentes, dans laquelle l'ester/les esters de phytostérol(s) est/sont un/des ester(s) d'acide(s) gras de stigmastérol, de sitostérol, de bêta-sitostérol, de brassicastérol, de campestérol, de 5-avénastérol et des isomères et des dérivés de ceux-ci.

9. Composition comestible selon l'une quelconque des revendications précédentes, dans laquelle le rapport molaire entre un/des diacylglycérol(s) et un/des ester(s) de phytostérol(s) et/ou de phytostanol dans ladite composition se trouve dans la plage allant de 1:5 à 5:1.

10. Composition comestible selon la revendication 9, dans laquelle le rapport molaire d'ester(s) de phytostérol(s) et/ou de phytostanol sur un/des diacylglycérol(s) dans ladite composition est de 2:1.

11. Composition comestible selon l'une quelconque des revendications précédentes, dans laquelle ladite composition est constituée de 15% en poids de DAG, principalement un/des 1,3-DAG(s) et de 25% en poids de PSE(s) total/totaux dissous ou dispersés dans une huile de poisson.

12. Composition comestible selon l'une quelconque des revendications précédentes, dans laquelle la composition comestible est une composition pharmaceutique pouvant être administrée par voie orale.

13. Composition comestible selon la revendication 12, dans laquelle la composition pharmaceutique pouvant être administrée par voie orale comprend en outre des additifs, des diluants, des excipients et/ou des vecteurs pharmaceutiquement acceptables.

14. Produit alimentaire ou complément alimentaire qui comprend une composition comestible selon l'une quelconque des revendications 1 à 13 pour une utilisation dans le traitement et la prévention de maladie cardio-vasculaire et de maladie coronarienne.

15. Produit alimentaire ou complément alimentaire selon la revendication 14, dans lequel la composition comestible comprend en outre des ingrédients conventionnels de compositions nutritionnelles.
